# EUROPEAN PATENT APPLICATION

(11) **EP 0 548 616 A1**
(43) Date of publication of application: **30.06.1993**
(21) Application number: 92120621.5
(22) Date of filing: 03.12.1992
(51) Int. Cl.: C07D 213/76, C09K 11/00

(54) **Aqueous chemiluminescent systems**

(30) Priority: 26.12.1991 US 813624
(71) Applicant: AMERICAN CYANAMID COMPANY, Stamford Connecticut 06904-0060 (US)
(72) Inventor: Steinmetz, Warren D., Havre de Grace, Maryland 21078 (US)
(74) Representative: Wächtershäuser, Günter, Prof. Dr.

(57) **Abstract**

Compounds having the formula: 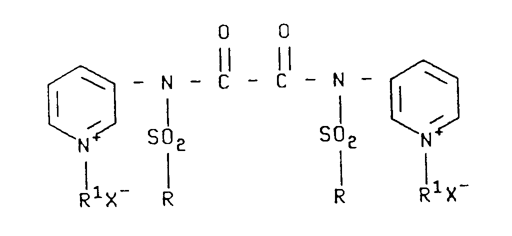
wherein R is a C₁-C₄ alkyl radical or an alkyl or halogen substituted phenyl radical, R¹ is a C₁-C₄ alkyl radical, and X is an anion, compositions containing the same and a method for creating chemiluminescence therewith, are disclosed.

## Description

### Background of The Invention

The art of generating light via chemical energy by the reaction of oxalic acid amides with a hydroperoxide in the presence of a fluorescer in aqueous systems is disclosed in U.S. Patent Nos. 4,282,357 and 4,338,213. However, there is an ever increasing demand for aqueous chemiluminescent compositions, which have higher light intensities and are more economically prepared than those set forth in these patents.

### Summary of The Disclosure

There is now provided a novel class of water-soluble amides of oxalic acid represented by the formula: 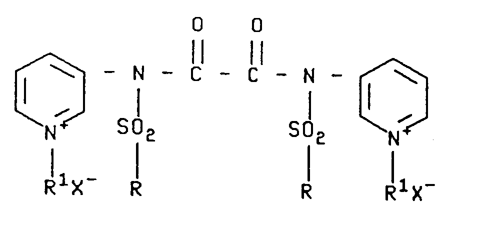
wherein R is a C₁-C₄ alkyl radical or a C₁-C₄ alkyl or halogen substituted phenyl radical, R¹ is a C₁-C₄ alkyl radical and X is an anion.

These compounds are very economically produced and when added to an agueous solution of water-soluble, organic fluorescer, especially one having a spectral emmission of 330-1000 nanometers, and hydrogen peroxide, or a source of hydrogen peroxide, result in the production of chemiluminescent emission of high intensity.

The anions of the compounds of the above-enumerated formula include the chloride, bromide, fluoride, methanesulfonate, methosulfate, trifluoromethane sulfonate, tetrafluoroborate and the like.

The preferred compounds of the above formula are those wherein the anion is the trifluoromethanesulfonate or methanesulfonate.

The novel oxamides of the present invention are produced by reacting 3-aminopyridine with an appopriately substituted sulfonyl chloride in the presence of a base such as triethylamine in a solvent such as toluene and at a temperature of 23^{o}C or below. The mixture is refuxed for 1/4 to 4 hours and then cooled to room temperature. The resultant solid is isolated and washed with water. The product is then treated with oxalyl chloride in a suitable solvent such is tetrahydrofuran at room temperature or below in the presence of base. The resultant reaction product is precipitated, for example, with hexane and then washed with water. The oxamide produced is then contacted with a methylating agent at room temperature in a solvent such as methylene chloride. The mixture resulting is refluxed for 1-6 hours and the product oxalic acid amide is recovered by filtration, etc.

The pyridinium positive charge plays a dual role in enhancing light output in chemiluminescent compositions containing the compounds of the above formula by enhancing the aqueous solubility and chemical reactivity with hydrogen peroxide.

The fluorescer compounds, useful in the chemiluminescent composition of this invention, may be defined broadly as water-soluble compounds, having an emission spectral maximum between 330 and 1,000 nanometers which do not react deleteriously with a hydrogen peroxide compound or the amide of oxalic acid on contact.

Numerous fluorescer compounds having the above-described properties are known. Many of these compounds are described in "Fluorescence and Phosphorescence" by Peter Pringsheim, Interscience Publishers, Inc., New York, New York, 1949 and in "dye Lasers" By F.P. Schafer, Editor, Springer Publishers, Berline, (1973). Others are described in "The Colour Index," Third Edition, Volume 4, the Society of Dyers and Colourists and The American Association of Textile Chemists and Colorists (1971).

The spectral distribution of the chemiluminescent emission from the composition of this invention is essentially the same as the fluorescence emission spectrum of the fluorescer compound employed.

Some specific examples of water-soluble fluorescer compounds of the class defined are as follows:
Sulfonated rubrene;
Rhodamine B(C.I. 45170);
Rhodamine 6G Perchlorate;
Sulforhodamine B(C.I. 45100);
Sulforhodamine 101;
9,10-Diphenylanthracene-2,6-disulfonic acid disodium salt;
3,9-Perylenedisulfonic acid disodium salt;
3,10-Perylenedisulfonic acid disodium salt;
8-Hydroxy-1,3,6-peyrenetrisulfonic acid trisodium salt;
Rhodamine 6G(C.I. 45160);
Disodium fluorescein (C.I. 45350:1);
Sulforhodamine G(C.I. 45220) and the like
The preferred water-soluble fluorescer is sulfonated rubrene.

The hydrogen peroxide compound employed in the composition and processes of this invention may be an aqueous solution of hydrogen peroxide per se, or a hydrogen peroxide-producing compound, such as sodium perborate potassium perborate, sodium carbonate peroxyhydrate, histidine perhydrate, and the like.

It has been found that the molar concentrations (moles per liter of solution) of the major components of the novel compositions, described herein, may vary considerably. It is only necessary that the components be present in sufficient concentration to obtain chemiluminescence. The molar concentration of the oxamide normally is in the range of 10-³ to 5, preferably about 10-² to 1.0. The molar concentration of the fluorescer compound used in from about 10-⁵ to about 10-¹, preferably 10-⁴ to 10-². The molar concentration of the hydrogen peroxide compound used is from about 10-³ to 10.0 preferably 10-¹ to 4.0. The optimum mole ratio of hydrogen peroxide compound to oxamide used ranges from about 0.1 to 10.0.

The ingredients of the chemiluminescent compositions of this invention are kept separated until chemiluminescence is desired, when they may be admixed in a single step or in a series of steps. The order of admixing of the ingredients is usually not critical. The hydrogen peroxide compound and fluorescer compound may be dissolved in water and the oxamide is added thereto to initiate chemiluminescence. The oxamide may be added as a solid or in a suitable diluent. Alternatively, the oxamide and the fluorescer compound may be dissolved in water and the hydrogen peroxide compound added in water and the hydrogen peroxide compound added thereto to initiate chemiluminescence. Preferably, the hydrogen peroxide compound in water is added to a solid mixture of oxamide and fluorescer to initiate chemiluminescence.

Superior intensity of chemiluminescence is obtained when the final mixture producing the luminescence is maintained at a temperature from about -10^{o} to 50^{o}C, preferably form about 15^{o} to 40^{o}C.

The invention is described in more detail by the following examples in which concentrations in moles per liter are indicated by the letter "M".

The examples are set forth for proposes of illustration only and are not to be construed as limitations on the instant invention except as set forth in the appended claims.

### Example 1

### Synthesis of oxalyl-bis-(N-methanesulfonyl)-bis-(N-3-aminomethylpyridinium)-methanesulfonate, (MAPQ-M).

### a. Synthesis of 3-Methanesulfonamidopyridine, (MAPS).

A suspension of 3-aminopyridine (100 parts, 1.06 mole) and triethylamine (129 parts, 1.28 mole) in toluene (dried over sodium) is warmed to 80^{o}C with vigorous stirring. Once the reaction becomes homogenous, it is cooled in an ice/water bath.
Methanesulfonyl chloride (1.27 mole) is then added dropwise to the cooled solution. After addition is completed, the reaction mixture is warmed to room temperature and finally heated at reflux temperature for 1.5 hours. The solution is allowed to cool slowly to room temperature with vigorous stirring. The solid reaction product and the by-product, triethylamine hydrochloride, are recovered by vacuum filtration. The solid is washed with water, and dried in a vacuum oven at 80^{o}C to give 155 parts of MAPS (85% yield): mp 125-134^{o}C; IR (KBr) 1320 and 1155 cm⁻¹.

### b. Synthesis of N,N'-bis-(3-aminopyridyl)-N,N'-bis(methanesulfonyl) oxamide, (MAPO).

Triethylamine (0.23 mole) is added to a suspension of MAPS, 3-methanesulfonamidopyridine (40 parts, 0.23 mole) in THF (freshly distilled over sodium/benzophenone). The mixture is cooled in an ice/water bath and kept under nitrogen atmosphere. Oxalyl chloride (0.115 mole, neat) is added dropwise to the stirred reaction mixture. After the addition is completed, the mixture is stirred for fifteen minutes. Hexane is added to the reaction mixture to precipitate the soluble product. The solid precipitate is collected on a filter, washed with 10% (w/v) aqueous sodium chloride solution followed by washing with water. The resulting solid material is dried under reduced pressure, yielding 36 parts of a tan solid (79% yield): mp 164-166^{o}C; IR (KBr) 1700, 1370, and 1160 cm⁻¹.

### c. Synthesis of oxalyl-bis-(N-methanesulfonyl)-bis-(N-3amino-methylpyridinium) methanesulfonate, (MAPQ-M).

Dimethylsulfate (9.263 mole) is added dropwise, at room temperature, to a stirred suspension of MAPO, N,N'-bis-(3-aminopyridyl)-N,N'-bis-(methanesulfonyl) oxamide (35 parts, 0.888 mole), in methylene chloride (dried over sieves). The reaction mixture is stirred at this temperature for one hour, followed by heating at reflux for three hours. The product is recovered by vacuum filtration, washed with methylene chloride and dried under reduced pressure to yield 50 parts of the titled product, an off-white solid, (87% yield): mp 159-167^{o}C IR (KBr) 1700, 1370, 1250, and 1180 cm⁻¹.

### Example 2

### Synthesis of oxalyl-bis-(N-methanesulfonyl)-bis-(N-3-aminomethylpyridinium) trifluoromethane sulfonate (MAPQ-T)

Methyl trifluoromethane sulfate (5.51 parts,) is drop added to a solution of MAPO, N,N'-bis-(3-aminopyridyl)-N,N'bis-(methanesulfonyl) oxamide (6.4 parts,) in methylene chloride (dried over sieves) at room temperature. After complete addition, stirring is continued for six hours at room temperature. The solid is recovered by filtration and vacuum dried to give 9.2 parts of MAPQ-T in 79% yield: mp 214-217^{o}C, IR (KBr) 1735, 1705, 1375, 1270 and 1170 cm⁻¹.

### Example 3

### Oxalyl-bis-(N-p-Toluenesulfonyl)-bis-(N-3-aminopyridinium trifluoromethane sulfonate, (TAPQ)

### a. Synthesis of 3-(p-tolunenesulfonamide) pyridine, (TAPS).

Para-toluenesulfonyl chloride (25.7 parts,) is added in small portions to a stirred solution of 3-aminopyridine (11.8 parts), triethylamine (18.2 parts) and pyridine (dried over potassium hydroxide). After complete addition, the reaction is stirred for 16 hours at room temperature then refluxed for one hour. The cooled solution is poured onto 500 parts of ice, stirred for one hour, and then filtered to give the crude product which is then recrystallized from a water/ethanol mixture using activated charcoal to give 13.0 parts of TAPS in 42% yield: mp 179-180^{o}C, (KBr) 1600, 1585, 1355, 1160 and 1090 cm⁻¹.

### b. Synthesis of N,N'-bis-(3-aminopyridyl)-N,N'-bis-(p-toluenesulfonyl) oxamide, (TAPO).

Oxalyl chloride (6.41 parts,) is drop-added to a stirred suspension of TAPS, 3-(p-toluenesulfonamido)pyridine (25 parts,) and triethylamine (10.1 parts,) in tetrahydrofuran (freshly distilled over sodium/benzophenone), cooled in an ice/water bath and kept under a nitrogen atmosphere. After addition is completed, the mixture is stirred for one-half hour in the ice/water bath and, then filtered to remove the triethylamine hydrochloride. The solvent was stripped from the filtrate and the resulting solid triturated in anhydrous ether. The insoluble material is removed by vacuum filtration and the filtrate concentrated to give 25.3 parts of TAPO in 92% yield: mp 180-183^{o}C, IR (KBr) 1690, 1370 and 1180 cm⁻¹.

### c. Synthesis of oxalyl-bis-(N-p-toluenesulfonly)-bis-(N-3-amino-methylpyridinium) trifluoremethane sulfonate (TAPQ).

Methyl trifluoromethane sulfate (11.8 parts,) is drop-added to a solution of TAPO, N,N'-bis-(3-aminopyridine)-N,N'bis-(p-toluenesulfonyl) oxamide (20 parts,) in methylene chloride (dried over sieves), cooled in an ice/water bath and kept under a nitrogen atmosphere. After complete addition, stirring is continued for two hours in the ice/water bath. The solid is recovered by filtration and vacuum dried to give 26.3 parts of TAPQ in 83% yield: mp 214-218^{o}C, IR (KBr) 1700, 1380, 1260 and 1170 cm⁻¹.

### Example 4

### Oxalyl-bis-(N-4-chlorophenylsulfonyl)-bis-(N-3-aminopyridinium) trifluoromethane sulfonate, (CAPQ)

### a. Synthesis of 3-(p-chlorophenyl-sulfonamido) pyridine, (CAPS).

A mixture of 3-aminopyridine (25 parts,) and p-chlorophenylsulfonyl chloride (56.1 parts,) in pyridine (dried over potassium hydroxide), is refluxed for four hours with vigorous stirring. The reaction is quenched with distilled water, cooled and the solid collected by vacuum filtration to gives the crude product. Recrystallization from water and ethanol gives 54.6 parts of CAPS in 77% yield: mp 185-186^{o}C, IR (KBr) 1575, 1480, 1350, 1320, and 1160 cm⁻¹.

### b. Synthesis of N,N'-bis-(3-aminopyridyl)-N,N'bis-(p-chlorophenylsulfonyl) oxamide, (CAPO).

Oxalyl chloride (8.3 parts) is drop-added to a stirred suspension of CAPO, 3-(p-chlorophenylsulfonamido)pyridinium (26.8 parts) and triethylamine (13.2 parts), in tetrahydrofuran (freshly distilled over sodium benzophenone), cooled in an ice/water bath, and kept under nitrogen atmosphere. After addition is completed, the mixture is stirred for one-half hour in the ice/water and then filtered to remove the solid triethylamine hydrochloride. The solvent is stripped from the filtrate to give 37.5 parts of CAPO in 98% yield: mp 186-189^{o}C, IR (KBr) 1 1370 and 1170 cm⁻¹.

### c. Synthesis of oxalyl-bis-(N-p-chlorophenylsulfonyl) -(N-3-aminomethylpyridinium) trifluoromethane sulfonate (CAPQ).

Methyl trifluoromethane sulfate (8.2 parts) is drop-added to a stirred solution of CAPO, N,N'bis-(3-aminopyridyl)-N,N'bis-(p-chlorophenylsulfonyl) oxamide (15 parts), in methylene chloride (dried over sieves), cooled in an ice/water bath and kept under a nitrogen atmosphere. After complete addition, stirring continued for two hours in the ice/water bath. The solid is recovered by vacuum filtration to give 22.5 parts of CAPO in 98% yield: mp 202-208^{o}C, IR (KBr) 1700, 1370, 1260 and 1170 cm⁻¹.

The reaction of 3-aminopyridine with the three cited sulfonating agents (methanesulfonyl chloride, toluenesulfonyl chloride and p-chlorophenylsulfonyl chloride) resulted in good yields (77-85%), with the exception of the toluenesulfonyl analog which realized a 42% yield (Table 1). The reaction of the isolated sulfonamides with oxalyl chloride was accomplished in very high yields (79-98%) and the methylation reactions using methylene trifluoromethane sulfate and dimethyl sulfate also proceeded in 79-98% yield. A compilation of yields (Table 1) is provided below. The various products were characterized by IR spectroscopy and, where appropriate, NM spectroscopy.

**Table I**

| Name | % Yields | | | 0 Average Yield |
|---|---|---|---|---|
| | Sulfonation | Oxalation | Methylation | |
| MAPQ-T | 85% | 79% | 79% | 53% |
| MAPQ-M | 85% | 79% | 87% | 58% |
| TAPQ | 42% | 92% | 93% | 32% |
| CAPQ | 77% | 98% | 98% | 74% |

All chemiluminescent experiments are performed using a chemiluminometer. The chemiluminometer is interfaced with a Bascom-Turner model 4110 recorder which graphically records and digitizes all data. Each chemiluminescence sample contains 0.23 part of sodium perborate tetrahydrate, 0.20 part of tartaric acid, 0.02 part of sodium decyl sulfate, 0.025 part of polyvinyl pyrrolidone - vinyl acetate copolymer and water. The concentration of the fluorescers are kept constant throughout, as follows: Rubrenesulfonate, 0.005 part; and 2-methyl-9,10-diphenylanthracenesulfonate, 0.005 part. It is important to note that chemiluminescence intensity curves are recorded immediately after the addition of water, for a sample collection time of 1500 seconds. Rubrenesulfonate samples are monitored at 575nm, and MDPAS samples are monitored at 440nm. The results are set forth in Table II, below.

**Table II**

| Example | Oximide | Area * |
|---|---|---|
| 5 | METQ | 1051.5 |
| 6 | MAPQ-M | 1573.5 |
| 7 | TAPQ | 3610.5 |
| 8 | CAPQ | 4890.0 |

| | | |
|---|---|---|
| * integration of the area under the curve of light output over time in relative units of luminance. | | |

One of the major goals, of this invention is to replace the presently used chemical initiator METQ with a more efficient and economical product. This has been accomplished by the development of the pyridinium derivatives of oxalic acid hereof. It is known that an efficient chemical initiator must possess a good leaving group to facilitate the attack of hydrogen peroxide. The compounds of this invention posess such a leaving group. Since the chemiluminescence light output is minimal in formulations which contain the unmethylated analogs of these compounds, the increase in chemiluminescence light output is believed to be due to a better leaving group ability by virtue of the quarternary pyridinium nitrogen. A good leaving group is present, therefore the ability to more efficiently produce energy releasing intermediates exists. Increasing the efficiency of production of energy releasing intermediates in turn increases the chemiluminescence efficiency.

The efficiencies of the production of the fluorescer excited states are equal for MAPQ and METQ, since the same high-energy intermediate are producing the fluorescer excited state. The fluorescer quantum yields are identical since the same environment is experienced by the fluorescer in both cases. Therefore, the increase in chemiluminescence efficiency observed for rubrenesulfonate may be because of a slight increase in the efficiency of the production of the energy releasing intermediate. The initiator MAPQ loses its sulfonamide group easier than does the initiator METQ.

The instant compounds have been found to emit bright chemiluminescence light output in the presence of the fluorescer rubrenesulfonate. This chemical initiator/fluorescer combination exhibits increased overall light output as compared to the previously used initiator, METQ. The light output of the instant compounds is about double that of METQ.

In addition to the positive results obtained for these instant compounds, it is also found that a greater chemiluminescence light output is observed in formulations which contain polymer, alone or in with formulations containing surfactant. This is true in the presence of either fluorescer rubrenesulfonate or 2-methyl-9,10-diphenylanthracenesulfonate when luminance measurements are used. In all cases, the best chemiluminescence light output is observed with a combination of both polymer and surfactant. Preferred polymers include polyvinylpyrolidone.

Following the procedures of Examples 1-4, various other amides of oxalic acid corresponding to the above formula are produced in accordance with the present invention. In each instance, the resultant amides preform in a manner analogous to those of this invention shown in Table II, above.

**Table III**

| Example | R | R¹ | X |
|---|---|---|---|
| 9 | p-methylphenyl | n-butyl | chloride |
| 10 | o-bromophenyl | ethyl | tetrafluoroborate |
| 11 | n-butyl | methyl | methosulfate |
| 12 | p-butylphenyl | t-butyl | methosulfonate |

## Claims

1. A water-soluble amide of oxalic acid represented by the formula: 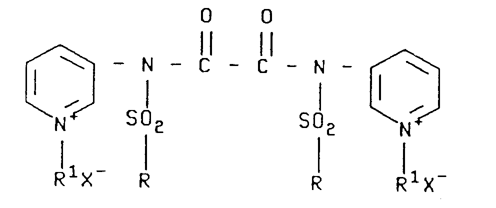 wherein R is a C₁-C₄ alkyl radical or a C₁-C₄ alkyl or halogen substituted phenyl radical, R¹ is a C₁-C₄ alkyl radical and X is an anion.

2. An amide according to Claim 1 wherein the anion is chloride, bromide, fluoride, methanesulfonate, methosulfate, trifluoromethanesulfonate or tetrafluoroborate.

3. An amide according to Claim 2 wherein R is methyl.

4. An amide according to Claim 3 wherein R¹ is methyl.

5. An amide according to Claim 4 wherein the anion is methanesulfate.

6. A composition for generating chemiluminescent emission comprising an aqueous solution of a water-soluble organic fluorescer having spectral emission in the range of 330-1000 nanometers and a water-soluble amide of oxalic acid represented by the formula: 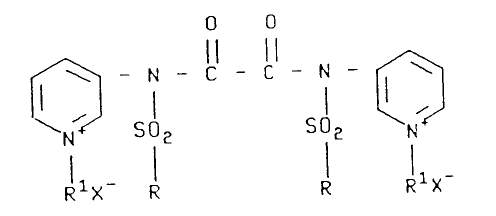 wherein R is a C₁-C₄ alkyl radical, or a C₁-C₄ alkyl or halogen substituted phenyl radical, R is a C₁-C₄ alkyl radical and X is an anion.

7. An amide according to Claim 6 wherein the anion is chloride, bromide, fluoride, methanesulfonate, methosulfate trifluoromethanesulfonate or tetrafluoroborate.

8. An amide according to Claim 7 wherein R is methyl.

9. An amide according to Claim 8 wherein R¹ is methyl.

10. An amide according to Claim 9 wherein the anion is methanesulfate.

11. A process for generating chemiluminescence comprising adding an effective amount of a water-soluble amide of oxalic acid represented by the formula: 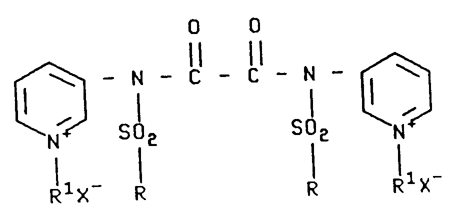 wherein R is a C₁-C₄ alkyl radical or a C₁-C₄ alkyl or halogen substituted phenyl radical, R¹ is a C₁-C₄ alkyl radical and X is an anion, into an aqueous solution of hydrogen peroxide, or a source of hydrogen peroxide, and a solid, water-soluble fluorescer having a spectral emission from about 330-1000 nanometers.

12. An amide according to Claim 11 wherein the anion is chloride, bromide, fluoride, methanesulfonate, methosulfate, trifluoromethanesulfonate or tetrafluoroborate.

13. An amide according to Claim 12 wherein R is methyl.

14. An amide according to Claim 13 wherein R¹ is methyl.

15. An amide according to Claim 14 wherein the anion is methanesulfate.
